# FASCICULE DE BREVET EUROPEEN

(11) **EP 0 976 382 B1**
(45) Date de publication et mention de la délivrance du brevet: **25.06.2003**
(21) Numéro de dépôt: 99113704.3
(22) Date de dépôt: 13.07.1999
(51) Int. Cl.: A61K 7/00, A61K 9/70

(54) **Patch cosmetique, pharmaceutique, ou dermatologique**
Kosmetisches oder dermopharmazeutisches Pflaster
Cosmetic or dermopharmaceutical patch

(30) Priorité: 30.07.1998 FR 9809794
(43) Date de publication de la demande: 02.02.2000
(73) Titulaire: L'OREAL, 75008 Paris (FR)
(72) Inventeur: Gueret, Jean-Louis H., 75016 Paris (FR)
(74) Mandataire: Schmit, Charlotte

(56) Documents cités:
- EP-A- 0 309 309
- WO-A-94/17837
- WO-A-94/22423
- WO-A-95/05204
- WO-A-95/28136
- WO-A-98/31315
- DE-A- 4 446 380
- US-A- 5 466 724

## Description

La présente invention a trait à un patch, cosmétique, pharmaceutique, ou dermatologique. L'invention vise tout particulièrement les patchs à action notamment, traitante, rafraîchissante, ou décontractante. De tels patchs ont une action cosmétique et/ou pharmaceutique sur la peau, par mise en contact avec la peau d'au moins une substance active dispersée dans une matrice. L'action, cosmétique et/ou pharmaceutique peut être obtenue rapidement (quelques minutes), ou résulter d'une mise en contact plus longue (par exemple, une heure ou plus).

L'invention vise tout particulièrement des patchs à forte teneur en eau, dans lesquels peuvent être incorporés différents actifs, et de ce fait, procurant une grande fraîcheur et une grande douceur à l'application. De tels patchs doivent pouvoir être manipulés aisément, notamment lors de la pose et de l'enlèvement.

Il est connu d'utiliser dans le domaine alimentaire, de la gomme de gellane y compris en mélange avec une autre gomme, en vue d'obtenir des produits gélifiés. Les conditions d'utilisation de la gomme de gellane pour ce type d'application, ne se prêtent pas à des applications cosmétiques, dermatologiques, ou pharmaceutiques, lesquelles applications nécessitent de bonnes propriétés à la fois de tenue, de solidité et de transfert.

Jusqu'ici les patchs cosmétiques, pharmaceutique, ou dermatologique à la portée du public sont décrits dans les documents suivants.

WO9528136 décrit un pansement comportant un système gélifiant composé d' un seul gélifiant . Ce système gélifiant comporte un polymère carboxy vinylique.

DE 44 46 380 présente un hydrogel contenant une substance active applicable sur la peau ainsi qu'un procédé d'application de l'hydrogel sur la peau qui présente un ensemble comprenant un compartiment pour contenir la composition contenant les différents ingrédients.

WO9505204 décrit un pansement constitué d'un matériel poreux comprenant des polymères hydrophilic. Ces polymères peuvent être synthétiques, biologiques ou particulièrement des polysaccharides .Mais l' hydrogel selon ce document ne comporte qu'un seul hydrocolloide, à savoir la gélatine.

En outre, un autre problème que se propose de résoudre au moins un mode de réalisation de l'invention, tient à la réalisation d'un patch, qui puisse être fabriqué à moindre coût, tout en autorisant des formes aussi variées que celles nécessitées par les applications cosmétiques, pharmaceutiques ou dermatologiques visées précédemment. Typiquement de tels patchs sont obtenus par découpe à la forme et aux dimensions voulues, d'une feuille constituée d'un support sur lequel est déposée puis séchée, une composition contenant un ou plusieurs actifs. Une telle réalisation fait intervenir des machines de couchage complexes, et génère des pertes importantes de matières premières, aussi bien en ce qui concerne le support que la composition contenant les actifs. De telles pertes sont d'autant plus importantes que la forme du patch n'est pas "simple" de manière à rendre ces patchs adaptés aux formes diverses et variées des différentes parties du visage, telles que le nez, le coin de l'oeil, le front, etc..

Enfin, une fois découpés, ces patchs doivent être conditionnés dans une pochette étanche, ce qui nécessite des manipulations supplémentaires, et donc augmente le coût de revient du patch.

Aussi, est-ce un des objets de l'invention que de réaliser un patch dans lequel sont résolus en tout ou partie, les problèmes discutés précédemment en référence à la technique antérieure.

C'est en particulier un objet de l'invention que de fournir un patch, pouvant être manipulé aisément, offrant à l'application sur la peau, des sensations nouvelles, notamment de fraîcheur et de douceur.

C'est un autre objet de l'invention que de réaliser un patch, formé directement in-situ, dans son conditionnement.

C'est encore un autre objet de l'invention que de réaliser un patch simple et économique à réaliser.

D'autres objets apparaîtront de manière détaillée dans la description qui suit.

Ces objets sont atteints en réalisant un patch cosmétique, pharmaceutique, ou dermatologique, comprenant une matrice formée à partir d'une composition (P) contenant, dans une phase aqueuse, un système gélifiant hydrophile, formé de l'association de gomme de gellane avec au moins un autre hydrocolloïde.

Le patch obtenu peut contenir une grande quantité d'eau, et est donc frais à l'application, tout en donnant une grande impression de douceur. Le patch peut être appliqué directement sur la peau, sans mouillage préalable de celle-ci. La composition formant la matrice est homogène et stable, et ne nécessite pas de technique de préparation particulière. Les compositions obtenues sont fraîches à l'application et permettent un bon transfert des actifs qu'elles contiennent, sur la peau, tout en étant suffisamment solides. La manipulation du patch, notamment sa pose ou son enlèvement, est aisée.

On entend par "patch" une structure sous forme d'une feuille ou nappe à une ou plusieurs couches, comprenant une matrice dans laquelle est incorporé au moins un actif, apte, lorsque la matrice est mise au contact de la peau à avoir une action sur la peau, soit par diffusion à l'intérieur de la peau (transdermie), soit par simple effet de contact de surface. Au bout d'un certain temps, le patch est retiré, sans qu'il n'y ait eu délitage de la matrice. Seul, tout ou partie de l'eau et des actifs contenus dans la matrice a disparu lors de l'application, notamment par évaporation ou transfert sur la peau. En fonction du mode d'action, le temps d'application varie dans une large mesure, et peut aller de quelques secondes à plusieurs heures, voire, plusieurs jours. La matrice peut être déposée sur un support apte à en favoriser la manipulation, et/ou à réaliser éventuellement une barrière occlusive. Une trame peut être intégrée à la matrice, soit en surface, soit noyée à l'intérieur de la matrice.

On entend par "hydrocolloïde" une macromolécule soluble dans l'eau, ne modifiant pas la valeur de l'activité en eau de la composition le contenant.

Avantageusement, le système gélifiant représente moins de 20% du poids total de la composition. La relativement faible proportion de gélifiants permet à la composition de ne pas laisser de dépôt visible lorsqu'elle est appliquée sur la peau. Par ailleurs, elle permet à la matrice, le cas échéant, d'être transparente ou translucide. Avantageusement, la matrice obtenue est sous forme solide, c'est à dire, ayant de préférence, une résistance à la compression supérieure ou égale à 20 grammes, à température ambiante (20-25°C), après pénétration par une sonde cylindrique de révolution ayant un diamètre de 0,8 cm dans la matrice de la composition dans une épaisseur de 1 mm à une vitesse de 0,5 mm/s et retrait de ladite sonde de la matrice de la composition à une vitesse de 0,5 mm/s ; la résistance à la compression étant mesurée avec un analyseur du type "LFRA Texture Analyser" commercialisé par la Société STEVENS/MECHTRIC.

De préférence, la gomme de gellane est présente en une quantité d'au moins 0,5 % du poids total de la composition. De préférence encore, la gomme de gellane est présente en une quantité allant de 2% à 15% et de préférence, de 2 à 6% du poids total de la composition.

L'hydrocolloïde associé à la gomme de gellane est choisi dans le groupe formé par : la cellulose ou ses dérivés ; les extraits d'algues ; les extraits de graines ; les exsudats de plantes ; les exsudats de micro-organismes ; les extraits de fruits ; les agents gélifiants d'origine animale ; les polymères synthétiques gélifiants hydrosolubles ; les polymères amphiphiles, les dérivés du silicium ; et leurs mélanges.

De manière plus détaillée, l'hydrocolloïde associé à la gomme de gellane peut être choisi dans le groupe formé par :
- la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les agents gélifiants d'origine animale tels que la gélatine, les caséïnates ;
- les polymères synthétiques gélifiants hydrosolubles tels que les acides polyacryliques réticulés, éventuellement par une chaîne alkyle, tels que les "Carbopol" ou " Pemulen" de la Société GOODRICH ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT,
- les polymères tels que le POLYCARE® commercialisé par la société Rhone-Poulenc sous la référence PS-20 ou PS-32,
- ou un mélange de ces composés.

Avantageusement, on utilise en association avec la gomme de gellane, un hydrocolloïde choisi parmi la gomme de caroube, la gomme de xanthane, les dérivés de cellulose, une gomme de guar modifiée et les mélanges de ces composés. Il s'agit plus particulièrement de la gomme de xanthane, de la carboxyméthylcellulose ou d'une gomme de guar modifiée. Cette dernière peut être notamment l'hydroxypropylguar.

De préférence, l'hydrocolloïde associé à la gomme de gellane est présent en une quantité allant de 0,5 à 10 % et de préférence de 0,5 à 5 % du poids total de la composition.

Avantageusement, la phase aqueuse représente de 60 à 97 % du poids total de la composition, et de préférence, 80 à 95 % du poids total de la composition.

Selon un mode particulier de l'invention, la composition comprend en outre au moins une huile, cette addition d'huile apportant un plus grand confort lors de l'application de la composition sur la peau.

Parmi les huiles utilisables, on peut citer les huiles minérales, les huiles d'origine végétale, les huiles d'origine animale, les huiles de synthèse telles que les esters gras, les huiles de silicone telles que les huiles de silicone volatile, les polyméthylsiloxanes, les polyméthylphénylsiloxanes, les polysiloxanes modifiés par des acides gras, des alcools gras ou des polyoxyalkylènes, les silicones fluorées et les huiles perfluorées. On peut ajouter d'autres corps gras tels que les acides gras, les alcools gras et les cires.

La ou les huiles et les autres corps gras éventuellement présents constituent la phase grasse.

La phase grasse peut être présente dans des proportions allant, par exemple, jusqu'à 30 %, de préférence de 0,1 à 20% et mieux de 0,5 à 10 % du poids total de la composition, ces proportions variant selon l'application choisie.

L'huile peut être introduite dans la phase aqueuse en présence d'un ou de plusieurs tensioactifs pour assurer une meilleure dispersion.

Les compositions selon l'invention peuvent donc également contenir un ou plusieurs tensioactifs non ioniques, anioniques, cationiques ou amphotères, habituellement utilisés dans les domaines cosmétique et/ou dermatologique. Lorsqu'il est présent, la quantité d'agent tensioactif va de préférence de 0,05 à 8 % et mieux de 0,05 à 5 % du poids total de la composition.

Il est possible de modifier la rigidité des compositions selon l'invention en y ajoutant un ou plusieurs sels qui vont augmenter cette rigidité. Ces sels peuvent être choisis parmi les sels des métaux mono-, di- ou trivalents, et plus particulièrement les sels de métal alcalin et alcalino-terreux et en particulier les sels de sodium et de calcium. Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate). La quantité de sel peut aller de 0,01 à 2 % et de préférence de 0,1 à 1 % du poids total de la composition. La rigidité peut être modifiée également par la présence d'au moins un alcool.

De préférence, le sel est choisi parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges. Des sels peuvent également être incorporés de manière à retarder la prise du gel, laquelle peut intervenir au bout d'une durée allant de 30 secondes à 24 heures.

La composition (P) peut contenir également, outre l'eau, au moins un solvant choisi parmi les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges. Ces solvants peuvent représenter 10 % en poids de la composition.

En outre, la composition (P) contient au moins un actif ayant un effet sur la peau, choisi notamment parmi les agents anti-oxydants, les anti-radicaux libres, les hydratants, les dépigmentants, les liporégulateurs, les anti-acnéiques, les anti-séborrhéiques, les agents anti-vieillissement, les adoucissants, les anti-rides, les kératolitiques, les anti-inflammatoires, les rafraîchissants, les cicatrisants, les protecteurs vasculaires, les anti-bactériens, les anti-fongiques, les anti-perspirants, les déodorants, les conditionneurs de la peau, les insensibilisants, les immunomodulateurs et nourrissants, ou les absorbeurs d'humidité (coton, polyacrylate), de sébum (Orgasol).

La composition (P) peut comporter au moins un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

Elle peut comporter également au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

La matrice peut être colorée de manière à permettre la visualisation des impuretés ou résidus prélevés sur la peau lors de l'application et/ou l'enlèvement du patch (11). Cette caractéristique est particulièrement avantageuse pour les patchs à action nettoyante, contenant par exemple des absorbeurs de sébum, dans la mesure où elle permet de renseigner l'utilisatrice sur la fréquence et la nature du traitement à réaliser.

La matrice peut être colorée par incorporation de pigments synthétiques, minéraux, ou organiques. Les pigments colorés peuvent être constitués notamment de pigments du type de ceux utilisés dans le domaine de l'alimentaire ou de la cosmétique, en particulier pour les rouges à lèvres ou les vernis à ongles. A titre d'exemple, on peut citer, seuls ou en combinaison, des pigments synthétiques, ou des pigments minéraux, notamment des pigments d'oxyde de zirconium ou de cérium, ainsi que les oxydes de fer ou de chrome, et le bleu ferrique. On peut utiliser des pigments organiques, notamment le noir de carbone, les laques de baryum, strontium, calcium (DC Red N°7), aluminium. A titre d'exemple plus spécifique, on utilise un pigment portant la référence DC violet 2 K7014, commercialisé par KOHNSTAMM®.

Le patch peut comporte également une trame, notamment sous forme d'un filet, d'un tissé ou d'un non tissé, ou d'un film perforé. La fonction de cette trame est multiple. Elle permet d'une part de conférer une plus grande intégrité au patch, et donc autorise la réalisation de patchs de plus faible épaisseur. En outre, elle facilite l'enlèvement du patch. La trame permet d'autre part de jouer sur la flexibilité du patch, et donc de permettre au patch de suivre parfaitement le profil de la surface sur laquelle il est appliqué.

Selon un premier mode de réalisation, le patch comprend un support sur lequel est enduite ladite composition, le support étant après enduction, découpé à la forme souhaitée. L'enduction à l'épaisseur souhaitée peut se faire au moyen d'un rouleau, d'une racle, ou par calandrage. Le support peut être constitué notamment d'un tissé ou non tissé, ou d'un film plastique perforé. Cette forme de réalisation du patch correspond à la technique conventionnelle de fabrication des patchs. Après découpe, le patch est ensuite conditionné à l'intérieur d'une pochette étanche.

Selon un autre aspect de l'invention, on réalise également un ensemble comprenant :
a) une coque définissant au moins un compartiment présentant un fond et dont un bord délimite, dans une face opposée audit fond, une première ouverture, obturée par un premier élément de fermeture amovible; et
b) un patch selon l'invention disposé de manière amovible dans le compartiment, ledit patch étant à la forme du compartiment, et obtenu par coulage dans ledit compartiment de ladite composition (P).

Ainsi, le patch est formé par moulage d'une composition dans une structure pouvant éventuellement être utilisée comme conditionnement final du patch, c'est à dire comme conditionnement dans lequel est commercialisé le patch. Les opérations et manipulations nécessaires à la fabrication de ce patch sont donc réduites. En outre, le patch peut être réalisé aisément, sous n'importe quelle forme géométrique, en fonction de la zone du corps ou du visage, à laquelle il est destiné. Avec ce mode de réalisation, il ne requiert aucune découpe, et n'engendre donc aucune perte de matière, aussi bien en support qu'en composition.

De préférence, la coque est formée par thermoformage ou injection à parois fines. La coque peut être formée par thermoformage ou injection à parois fines. On utilise de préférence un matériau ou un complexe de matériaux, étanche à l'oxygène et à la vapeur d'eau. A titre préférentiel, la coque est formée d'un matériau thermoplastique choisi parmi les polyéthylènes, ou les polypropylènes, ou d'un complexe de matériaux thermoplastiques, tel que le Polyéthylène Téréphtalate/Aluminium/Polyéthylène, ou les élastomères, notamment les élastomères de silicone. La réalisation de la coque en un matériau élastiquement déformable permet de faciliter le démoulage du patch, notamment en vue de son utilisation.

Avantageusement, la coque forme un rebord tout autour de ladite première ouverture, de manière à y permettre la fixation, notamment par collage, thermoscellage ou soudure, du premier élément de fermeture.

Le premier élément de fermeture peut être constitué d'un opercule, formé d'un matériau métallique (Aluminium) ou thermoplastique tel qu'un polyéthylène, un polypropylène, ou d'un complexe de tels matériaux. On assure ainsi une parfaite conservation du patch ainsi conditionné. L'enlèvement d'un tel opercule peut être facilité par la présence d'un "coin cassé" formé par la coque, et auquel est collé l'opercule, de manière à en faciliter l'enlèvement par arrachage.

Alternativement, la composition est coulée dans le compartiment par le fond. Dans cette hypothèse, le compartiment comprend au moins une seconde ouverture, ménagée dans ledit fond, ladite matrice étant moulée dans le compartiment, par coulage via ladite seconde ouverture, de ladite composition, ladite seconde ouverture étant obturée après remplissage du compartiment. L'élément de fermeture amovible obturant la première ouverture, est, dans ce cas de figure, mis en place avant coulage de la composition par le fond.

La seconde ouverture peut être obturée par un opercule, collé ou soudé autour de ladite seconde ouverture.

Dans le mode de réalisation d'un patch avec trame, celle-ci peut être disposée au voisinage d'une première face du patch opposée au fond du compartiment. Tel peut être le cas lorsque la composition est coulée notamment par le fond. Alternativement, la trame est située, au voisinage d'une seconde face du patch adjacente au fond du compartiment. Dans ce cas, la composition est coulée par le dessus, en ayant, préalablement au coulage, déposé ladite trame dans le fond du compartiment. Alternativement encore, la trame est noyée dans la matrice, dans une position située entre la première et la seconde face. A cet effet, on peut couler la composition par le dessus. On forme alors une première couche de ladite composition dans le fond du compartiment. On dépose la trame sur la première couche, et on coule ensuite une deuxième couche de ladite composition.

La composition peut être coulée dans le compartiment à une température de l'ordre de 80 °C à 90 °C, le figeage ou gélification de la composition sous forme d'un gel se produisant aux environs de 60 à 70 °C. Dans la pratique, bien que ne constituant pas un mode préférentiel, la composition peut être coulée en cours de figeage. Le figeage peut se produire lors du refroidissement ou par réaction avec l'eau contenue dans la composition.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 représente un premier mode de réalisation d'un ensemble formé d'un conditionnement dans lequel est disposé un patch selon l'invention;
- les figures 2A-2C illustrent un premier mode de remplissage de l'ensemble de la figure 1;
- les figures 3A-3C illustrent des variantes du procédé des figures 2A-2C; et
- les figures 4A-4C illustrent un second mode de remplissage de l'ensemble de la figure 1.

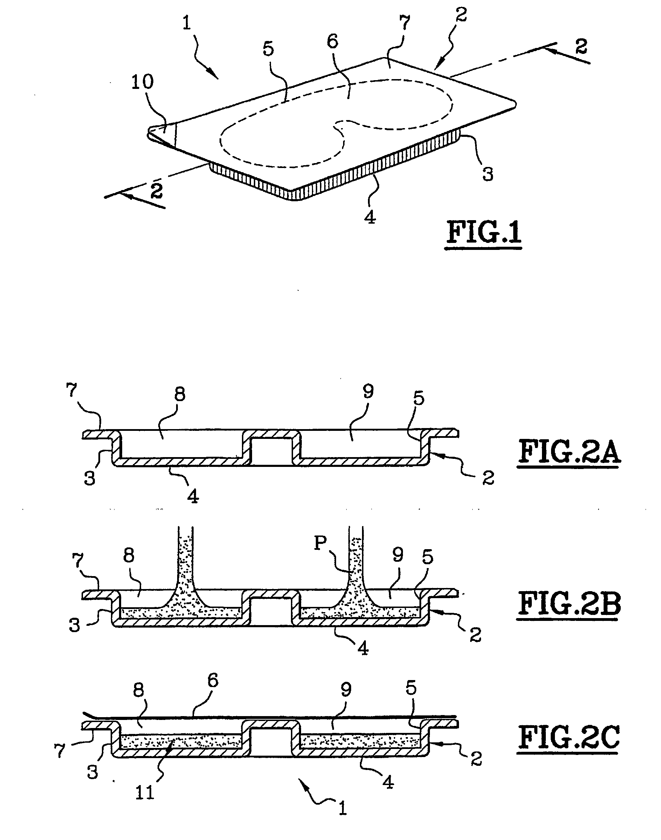

A la figure 1, l'ensemble 1 représenté comprend une coque 2 obtenue par thermoformage ou injection à parois fines d'un matériau tel qu'un polypropylène. La coque 2 forme un compartiment en creux 3 dont la forme correspond à celle d'un masque pour les yeux. Ledit compartiment présente un bord délimitant sur sa face opposée au fond 4, une ouverture 5, obturée par un opercule thermoscellé 6. L'opercule thermoscellé 6 est formé d'un complexe Polypropylène/Aluminium/Polyéthylène. A cet effet, la coque forme un rebord 7 tout autour dé l'ouverture 5, de manière à permettre la fixation de l'opercule 6, et ce, de manière étanche tout autour de l'ouverture 5. La coque 2 présente un coin cassable 10, permettant de favoriser l'enlèvement de l'opercule 6, en vue de l'utilisation d'un patch cosmétique contenu à l'intérieur du compartiment 3, ledit patch étant, comme on le verra plus en détail par la suite, formé directement in-situ, à l'intérieur du compartiment 3.

Les figures 2A-2C illustrent une vue en coupe selon la ligne 2A-2C de l'ensemble 1 de la figure 1. A la figure 2A, la coque 2 est posée sur son fond, l'ouverture 5 n'étant pas obturée. A la figure 2B, la composition liquide P apte à former la matrice gélifiée du patch est coulée au moyen de deux becs situés en regard de chacune des deux zones 8, et 9 du compartiment 3. Dans cette phase de coulage, la température de la composition liquide P est de l'ordre de 80 °C à 90 °.

Lors du refroidissement de la composition P, la composition liquide se fige de manière à former un patch gélifié 11 à la forme et aux dimensions (hormis la profondeur) du compartiment 3. Typiquement, le figeage se produit à une température qui peut être de l'ordre de 60 °C à 70 °C. Avant, pendant, ou après le figeage de la composition, un opercule 6 est thermoscellé sur la coque 2, de manière à obturer l'ouverture 5.

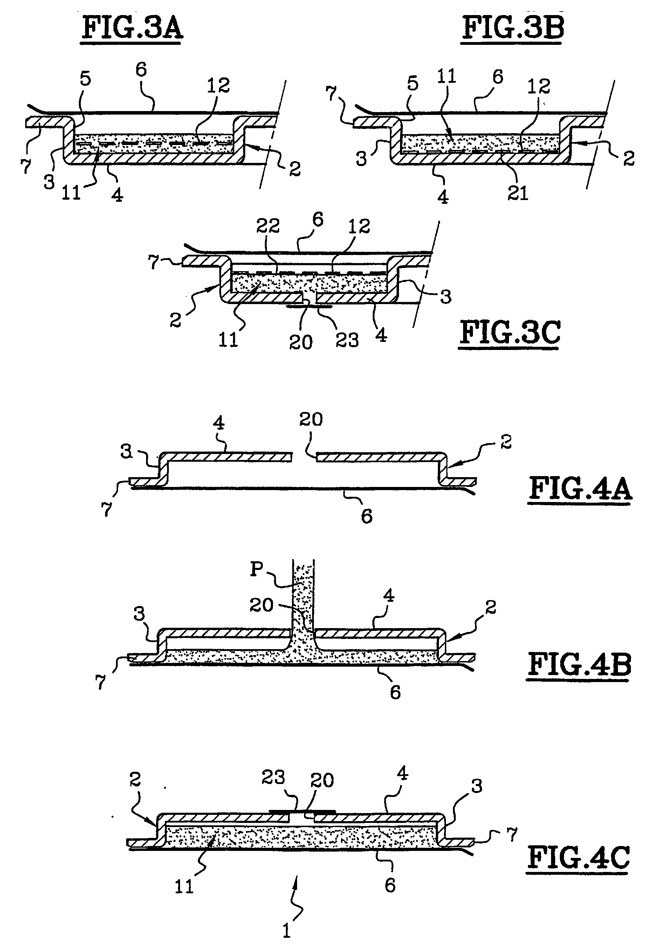

Les figures 3A-3C illustrent des variantes du mode de réalisation précédent. A la figure 3A, une trame 12, formée d'un filet de polyamide, est noyée dans la matrice du patch 11. Pour ce faire, on a procédé au coulage de la composition, au travers de l'ouverture 5, et ce en deux étapes successives. Dans une première étape, une première couche de ladite composition P est déposée dans le fond du compartiment 3. La trame 12 est ensuite déposée à la surface de cette première couche. Une seconde couche de ladite composition est ensuite déposée sur la trame 12. La trame permet de réduire l'épaisseur du patch tout en conservant une intégrité suffisante. L'épaisseur du patch peut aller de quelques 10èmes de mm à quelques mm, en fonction de l'application souhaitée. La trame peut avoir une épaisseur comprise entre 0,01 mm et 2 mm. Son poids peut aller de 5 à 60 g/m², et de préférence, de 10 à 40 g/m².

Dans le mode de réalisation de la figure 3B, la trame 12 est déposée dans le fond 4 du compartiment 3 avant le coulage de la composition de sorte que ladite trame soit située au voisinage de la face 21 du patch 11 adjacente au fond 4.

Dans le mode de réalisation de la figure 3C, le coulage de la composition est effectué, comme on le verra plus en détail par la suite, via une ouverture 20, ménagée dans le fond 4 du compartiment 3. L'opercule 6 est thermoscellé sur l'ouverture 5 avant le coulage de la composition, et de ce fait, fait office de fond pour le coulage de la composition. Avant coulage, une trame 12 est déposée à l'intérieur du compartiment. Dans la pratique, la trame est disposée dans le compartiment 3, avant scellage de l'ouverture 5. En retournant l'ensemble, la trame 12 vient se positionner en contact de l'opercule 6. Ainsi, dans le patch final, la trame 12 est située au voisinage d'une face 22 du patch 11 située en regard de l'opercule thermoscellé 6. Après coulage de la composition, et de préférence, avant figeage, l'ouverture 20 est obturée par un opercule-23 collé ou thermoscellé. L'ensemble 1 est ensuite retourné de sorte que le figeage de la matrice se fasse en contact du fond 4 du compartiment 3.

Les figures 4A-4C illustrent une variante du mode de remplissage des figures 2A-2C, et selon laquelle la composition est coulée dans le compartiment 3 via une ouverture 20 ménagée dans le fond 4 du compartiment. A la figure 4A, le compartiment 3, dont l'ouverture 5 a été préalablement scellée par l'opercule 6, est retourné tête en bas. A la figure 4B, la composition P est coulée dans le compartiment 3, via l'ouverture 20 ménagée dans le fond 4 du compartiment 3. Une seconde ouverture (non représentée) peut être prévue dans le fond du compartiment de manière à autoriser l'échappement d'air lors du coulage à chaud. A la figure 4C, l'ouverture 20 est scellée au moyen d'une pastille adhésive 23, collée ou thermoscellée. De préférence, l'ensemble 1 est retourné avant figeage de la composition liquide P.

Pour utiliser le patch selon l'invention, l'utilisatrice enlève l'opercule 6. En provoquant une légère flexion du fond 4 du compartiment 3, elle démoule le patch 11, et le prend avec les doigts pour l'appliquer sur la partie correspondante du visage ou du corps. A cet effet, le patch peut être de formes diverses. Il peut être notamment adapté à la forme des yeux, du front, du nez, du tour de la bouche, etc. Le démoulage du patch 11 peut être favorisé en réalisant la barquette 2 en matériau élastiquement déformable, notamment en élastomère thermoplastique.

### EXEMPLE 1 :

Dans 180 g d'eau, on incorpore :
- 3% en poids de gomme de gellane;
- 1% en poids de gomme de xanthane;
- 1% de germe de blé;
- 0,2% de conservateur;
- 2% d'orgasol; et
- 0,15% d'huile essentielle de lavande.

La composition est coulée à une température de 90° dans une barquette thermoformée en polypropylène. La composition est coulée dans le fond de la barquette sur une épaisseur de 1 mm, un filet de polyamide étant préalablement déposé dans le fond de la barquette. Après coulage, la barquette est refermée par un opercule thermoscellé.

Après démoulage par l'utilisatrice, le patch est appliqué sur le visage pendant 5 mn à 60 mn. Un tel patch a une action décontractante et tenseur, apaisante.

### EXEMPLE 2 :

Dans 180 g d'eau, on incorpore :
- 2,5% en poids de gomme de gellane;
- 0,7% en poids de gomme de xanthane;
- 1% d'alginate;
- 0,5% d'acide ascorbique;
- 0,5% de conservateur;
- 2% d'éthanol; et
- 2% de SPF.

La composition est coulée à une température de 90° dans une barquette injectée en Polyéthylène. La composition est coulée dans le fond de la barquette sur une épaisseur de 1 mm, un filet de polyamide étant préalablement déposé dans le fond de la barquette. Après coulage, la barquette est refermée par un opercule thermoscellé.

Après démoulage par l'utilisatrice, le patch est appliqué sur-le visage pendant 5 mn à 60 mn. Un tel patch a une action apaisante, éclaircissante, et nivelante.

### EXEMPLE 3 :

Dans 180 g d'eau, on incorpore :
- 2,5% en poids de gomme de gellane;
- 0,7% en poids de gomme de xanthane;
- 2% d'extrait de fleur;
- 0,5% d'éthanol; et
- 0,5% de cristaux de menthe.

La composition est coulée à une température de 90° dans une barquette thermoformée, obtenue d'un complexe Polyéthylène téréphtalate/Aluminium/Polyéthylène. La composition est coulée dans le fond de la barquette sur une épaisseur de 1 mm, un filet de polyamide étant préalablement déposé dans le fond de la barquette. Après coulage, la barquette est refermée par un opercule thermoscellé.

Après démoulage par l'utilisatrice, le patch est appliqué sur le visage pendant 5 mn à 60 mn. Un tel patch a une action rafraîchissante et aseptisante.

Dans la description détaillée qui précède, il a été fait référence à des modes de réalisation préférés de l'invention.

## Revendications

1. Patch cosmétique, pharmaceutique, ou dermatologique, comprenant une matrice formée à partir d'une composition (P) contenant, dans une phase aqueuse, un système gélifiant hydrophile, formé de l'association de gomme de gellane avec au moins un autre hydrocolloïde.

2. Patch cosmétique selon la revendication 1 **caractérisé en ce que** le système gélifiant représente moins de 20% du poids total de la composition (P).

3. Patch selon la revendication 1 ou 2 **caractérisé en ce que** la gomme de gellane est présente en une quantité d'au moins 0,5 % du poids total de la composition (P).

4. Patch selon l'une quelconque des revendications 1 à 3 **caractérisé en ce que** la gomme de gellane est présente en une quantité allant de 2% à 15% et de préférence, de 2 à 6% du poids total de la composition (P).

5. Patch selon l'une quelconque des revendications 1 à 4, **caractérisé en ce que** l'hydrocolloïde associé à la gomme de gellane est choisi dans le groupe formé par : la cellulose ou ses dérivés ; les extraits d'algues ; les extraits de graines ; les exsudats de plantes ; les exsudats de micro-organismes, telle que la gomme de xanthane ; les extraits de fruits ; les agents gélifiants d'origine animale ; les polymères synthétiques gélifiants hydrosolubles ; le POLYCARE®, les dérivés du silicium ; et leurs mélanges.

6. patch selon l'une quelconque des revendications 1 à 5, **caractérisé en ce que** l'hydrocolloïde associé à la gomme de gellane est choisi dans le groupe formé par la gomme de xanthane, les dérivés de cellulose, la gomme de caroube, une gomme de guar modifiée et leurs mélanges.

7. Patch selon l'une quelconque des revendications précédentes, **caractérisé en ce que** l'hydrocolloïde associé à la gomme de gellane est présent en une quantité allant de 0,5 à 10 % et de préférence de 0,5 à 5 % du poids total de la composition (P).

8. Patch selon l'une quelconque des revendications précédentes, **caractérisé en ce que** la phase aqueuse représente de 60 à 97 % du poids total de la composition (P).

9. Patch selon l'une quelconque des revendications précédentes, **caractérisé en ce qu'**elle comprend en outre au moins une phase grasse.

10. Patch selon la revendication précédente, **caractérisée en ce que** la phase grasse est présente en une quantité allant jusqu'à 30 % et de préférence de 0,1 à 20% du poids total de la composition.

11. Patch selon l'une quelconque des revendications précédentes **caractérisé en ce que** la composition (P) contient un solvant choisi parmi les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

12. Ensemble selon l'une quelconque des revendications précédentes, **caractérisé en ce que** la composition (P) contient au moins un actif ayant un effet sur la peau, choisi notamment parmi les agents anti-oxydants, les anti-radicaux libres, les hydratants, les dépigmentants, les liporégulateurs, les anti-acnéiques, les anti-séborrhéiques, les agents anti-vieillissement, les adoucissants, les anti-rides, les kératolitiques, les anti-inflammatoires, les rafraîchissants, les cicatrisants, les protecteurs vasculaires, les anti-bactériens, les anti-fongiques, les anti-perspirants, les déodorants, les conditionneurs de la peau, les insensibilisants, les immunomodulateurs et nourrissants, ou les absorbeurs d'humidité (coton, polyacrylate), de sébum (Orgasol).

13. Patch selon la revendication 12 **caractérisé en ce que** la composition (P) comporte au moins un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

14. Patch selon la revendication 12 **caractérisé en ce que** la composition (P) comporte au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de famesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

15. Patch selon l'une quelconque des revendications 1 à 14 **caractérisé en ce que** la composition (P) contient au moins un composé retardateur de prise, notamment sous forme d'au moins un sel.

16. Patch selon l'une quelconque des revendications précédentes **caractérisé en ce que** la matrice est colorée de manière à permettre la visualisation des impuretés ou résidus prélevés sur la peau lors de l'application et/ou l'enlèvement du patch.

17. Patch selon la revendication 16 **caractérisé en ce que** la matrice est colorée par incorporation de pigments synthétiques, minéraux, ou organiques.

18. Patch selon l'une quelconque des revendications 1 à 17 **caractérisé en ce que** le patch comporte également une trame (12), notamment sous forme d'un filet, d'un tissé ou d'un non tissé, ou d'un film perforé.

19. Patch selon l'une quelconque des revendications précédentes, **caractérisé en ce qu'**il comprend un support sur lequel est enduite ladite composition, le support étant après enduction, découpé à la forme souhaitée.

20. patch selon la revendication 19 **caractérisé en ce que** le support est constitué notamment d'un tissé ou non tissé, ou d'un film plastique perforé.

21. Ensemble (1) comprenant :
a) une coque (2) définissant au moins un compartiment (3) présentant un fond (4) et dont un bord délimite, dans une face opposée audit fond, une première ouverture (5), obturée par un premier élément de fermeture amovible (6); et
b) un patch (11) selon l'une quelconque des revendications précédentes, disposé de manière amovible à l'intérieur du compartiment (3), ledit patch (11) étant à la forme du compartiment (3), et obtenu par coulage dans ledit compartiment (3) de ladite composition (P).

22. Ensemble selon la revendication 21 **caractérisé en ce que** la coque (2) est formée par thermoformage ou injection à parois fines.

23. Ensemble selon la revendication 22 **caractérisé en ce que** la coque (2) est formée d'un matériau thermoplastique choisi parmi les polyéthylènes, ou les polypropylènes, ou d'un complexe de matériaux thermoplastiques, tel que le Polyéthylène Téréphtalate/Aluminium/Polyéthylène, ou les élastomères, notamment les élastomères de silicone.

24. Ensemble selon l'une quelconque des revendications 21 à 23 **caractérisé en ce que** la matrice est moulée dans ledit compartiment (3), par coulage via ladite première ouverture (5), de ladite composition (P).

25. Ensemble selon l'une quelconque des revendications 21 à 24 **caractérisé en ce que** la coque- (2) forme un rebord (7) tout autour de ladite première ouverture (5), de manière à y permettre la fixation, notamment par collage, thermoscellage ou soudure, du premier élément de fermeture (6).

26. Ensemble selon l'une quelconque des revendications 21 à 25 **caractérisé en ce que** le premier élément de fermeture (6) est constitué d'un opercule, formé d'un matériau métallique ou thermoplastique tel qu'un polyéthylène, un polypropylène, ou d'un complexe de tels matériaux.

27. Ensemble selon l'une quelconque des revendications 21 à 26 **caractérisé en ce qu'**il comprend au moins une seconde ouverture (20), ménagée dans ledit fond (4), ladite matrice étant moulée dans le compartiment (3), par coulage de ladite composition (P) via ladite seconde ouverture (20), ladite seconde ouverture (20) étant obturée après remplissage du compartiment.

28. Ensemble selon la revendication 27 **caractérisé en ce que** ladite seconde ouverture (20) est obturée par un opercule (23), collé ou soudé autour de ladite seconde ouverture.

29. Ensemble selon l'une quelconque des revendications 21 à 28 **caractérisé en ce qu'**une trame (12) est disposée au voisinage d'une première face (22) du patch (11) opposée au fond (4) du compartiment, ou au voisinage d'une seconde face (21) du patch adjacente au fond (4) du compartiment (3), ou noyée dans la matrice, dans une position située entre la première (22) et la seconde face (21).

## Patentansprüche

1. Kosmetisches, pharmazeutisches oder dermatologisches Pflaster, das eine Matrix aufweist, die aus einer Zusammensetzung (P) gebildet ist, die in einer wässerigen Phase ein hydrophiles gelierendes System enthält, das von der Kombination von Gellangummi mit mindestens einem weiteren Hydrokolloid gebildet wird.

2. Kosmetisches Pflaster nach Anspruch 1, **dadurch gekennzeichnet, dass** das gelierende System weniger als 20 % des Gesamtgewichts der Zusammensetzung (P) ausmacht.

3. Pflaster nach Anspruch 1 oder 2, **dadurch gekennzeichnet, dass** das Gellangummi in einem Mengenanteil von mindestens 0,5 % des Gesamtgewichts der Zusammensetzung (P) vorliegt.

4. Pflaster nach einem der Ansprüche 1 bis 3, **dadurch gekennzeichnet, dass** das Gellangummi in einem Mengenanteil von 2 bis 15 % und vorzugsweise von 2 bis 6 % des Gesamtgewichts der Zusammensetzung (P) vorliegt.

5. Pflaster nach einem der Ansprüche 1 bis 4, **dadurch gekennzeichnet, dass** das mit dem Gellangummi kombinierte Hydrokolloid unter Cellulose oder ihren Derivaten, Algenextrakten, Samenextrakten, Exsudaten von Pflanzen, Exsudaten von Mikroorganismen, wie Xanthangummi, Fruchtextrakten, Gelbildnern tierischen Ursprungs, wasserlöslichen gelierenden synthetischen Polymeren, POLYCARE®, Siliciumderivaten und den Gemischen dieser Verbindungen ausgewählt ist.

6. Pflaster nach einem der Ansprüche 1 bis 5, **dadurch gekennzeichnet, dass** das mit dem Gellangummi kombinierte Hydrokolloid unter Xanthangummi, Cellulosederivaten, Johannisbrotkernmehl, einem modifizierten Guargummi und den Gemischen dieser Verbindungen ausgewählt ist.

7. Pflaster nach einem der vorhergehenden Ansprüche, **dadurch gekennzeichnet, dass** das mit dem Gellangummi kombinierte Hydrokolloid in einem Mengenanteil von 0,5 bis 10 % und vorzugsweise von 0,5 bis 5 % des Gesamtgewichts der Zusammensetzung (P) vorliegt.

8. Pflaster nach einem der vorhergehenden Ansprüche, **dadurch gekennzeichnet, dass** die wässerige Phase 60 bis 97 % des Gesamtgewichts der Zusammensetzung (P) ausmacht.

9. Pflaster nach einem der vorhergehenden Ansprüche, **dadurch gekennzeichnet, dass** es ferner mindestens eine Fettphase aufweist.

10. Pflaster nach dem vorhergehenden Anspruch, **dadurch gekennzeichnet, dass** die Fettphase in einem Mengenanteil von bis zu 30 % und vorzugsweise von 0,1 bis 20 % des Gesamtgewichts der Zusammensetzung vorliegt.

11. Pflaster nach einem der vorhergehenden Ansprüche, **dadurch gekennzeichnet, dass** die Zusammensetzung (P) ein Lösemittel enthält, das unter primären Alkoholen, wie Ethanol und Isopropanol, Glykolen, wie Propylenglykol, Butylenglykol, Dipropylenglykol und Diethylenglykol, Glykolethern, wie C₁₋₄-Alkylethern von Mono-, Di- oder Tripropylenglykol oder Mono-, Di- oder Triethylenglykol, und den Gemischen dieser Verbindungen ausgewählt ist.

12. Einheit nach einem der vorhergehenden Ansprüche, **dadurch gekennzeichnet, dass** die Zusammensetzung (P) mindestens einen Wirkstoff enthält, der eine Wirkung auf die Haut aufweist und der insbesondere unter Antioxidantien, Radikalfängern für freie Radikale, Hydratisierungsmitteln, depigmentierenden Wirkstoffen, Lipidregulatoren, Wirkstoffen gegen Akne, Antiseborrhoeika, Wirkstoffen gegen Alterung, reizlindernden Wirkstoffen, Wirkstoffen gegen Falten, Keratolytika, entzündungshemmenden Wirkstoffen, erfrischenden Wirkstoffen, Wirkstoffen für die Wundheilung, vaskulären Schutzmitteln, antibakteriellen Wirkstoffen, Antimykotika, Antiperspirantien, Deodorants, Hautkonditionierern, Anästhetika, Immunmodulatoren, nährenden Wirkstoffen oder Feuchtigkeitsabsorbern (Baumwolle, Polyacrylat) und Talgabsorbern (Orgasol) ausgewählt ist.

13. Pflaster nach Anspruch 12, **dadurch gekennzeichnet, dass** die Zusammensetzung (P) mindestens einen wasserlöslichen Wirkstoff aufweist, der unter den folgenden Verbindungen ausgewählt ist: Ascorbinsäure und ihren biologisch kompatiblen Salzen, Enzymen, Antibiotika, Komponenten mit straffender Wirkung, α-Hydroxysäuren und ihren Salzen, Polyhydroxycarbonsäuren, Saccharosen und ihren Derivaten, Harnstoff, Aminosäuren, Oligopeptiden, wasserlöslichen Pflanzenextrakten und Hefeextrakten, Proteinhydrolysaten, Hyaluronsäure, Mucopolysacchariden, den Vitaminen B₂, B₆, H und PP, Panthenol, Folsäure, Acetylsalicylsäure, Allantoin, Glycyrrhetinsäure, Kojisäure und Hydrochinon.

14. Pflaster nach Anspruch 12, **dadurch gekennzeichnet, dass** die Zusammensetzung (P) mindestens eine fettlösliche Verbindung aufweist, die unter den folgenden Verbindungen ausgewählt ist: D-α-Tocopherol, DL-α-Tocopherol, D-α-Tocopherylacetat, DL-α-Tocopherylacetat, Ascorbylpalmitat, Vitamin F und Glyceriden von Vitamin F, D-Vitaminen, Vitamin D₂, Vitamin D₃, Retinol, Retinolestern, Retinolpalmitat, Retinolpropionat, β-Carotin, D-Panthenol, Farnesol, Farnesylacetat, Jojobaöl und Öl von schwarzen Johannisbeeren, die reich an essentiellen Fettsäuren sind, Keratolytika, wie Salicylsäure, ihren Salzen und ihren Estern, 5-(n-Octanoyl)-salicylsäure und ihren Estern, Alkylestern von α-Hydroxysäuren, wie Citronensäure, Milchsäure und Glykolsäure, Asiatsäure, Madecassischer Säure, Asiaticosid, dem Totalextrakt von Centella asiatica, β-Glycyrrhetinsäure, α-Bisabolol, Ceramiden, wie 2-Oleoylamino-1,3-octadecan, Phytantriol, Sphingomyclin von Milch, Phospholipiden marinen Ursprungs, die reich an mehrfach ungesättigten essentiellen Fettsäuren sind, Ethoxychin, Rosmarinextrakt, Melisseextrakt, Quercetin, dem Extrakt von getrockneten Mikroalgen und steroidalen entzündungshemmenden Wirkstoffen.

15. Pflaster nach einem der Ansprüche 1 bis 14, **dadurch gekennzeichnet, dass** die Zusammensetzung (P) mindestens eine Verbindung insbesondere in Form mindestens eines Salzes zur Verzögerung des Gelierens enthält.

16. Pflaster nach einem der vorhergehenden Ansprüche, **dadurch gekennzeichnet, dass** die Matrix gefärbt ist, um Unreinheiten oder Rückstände sichtbar machen zu können, die bei der Anwendung und/oder beim Abnehmen des Pflasters von der Haut entfernt werden.

17. Pflaster nach Anspruch 16, **dadurch gekennzeichnet, dass** die Matrix durch Einarbeiten von synthetischen, anorganischen oder organischen Pigmenten gefärbt ist.

18. Pflaster nach einem der Ansprüche 1 bis 17, **dadurch gekennzeichnet, dass** das Pflaster auch eine Lage (12) insbesondere in Form eines Netzes, eines gewebten Stoffs oder eines Vliesstoffs oder in Form einer perforierten Folie aufweist.

19. Pflaster nach einem der vorhergehenden Ansprüche, **dadurch gekennzeichnet, dass** es einen Träger aufweist, auf den die Zusammensetzung aufgetragen wird, wobei der Träger nach dem Beschichten auf die gewünschte Form zugeschnitten wird.

20. Pflaster nach Anspruch 19, **dadurch gekennzeichnet, dass** der Träger insbesondere aus einem gewebten Stoff, einem Vliesstoff oder einem perforierten Kunststoff besteht.

21. Einheit (1), die umfasst:
(a) eine Schale (2), die mindestens ein Kompartiment (3) abgrenzt, das einen Boden (4) und auf einer dem Boden gegenüber liegenden Seite einen Rand aufweist, der eine erste Öffnung (5) begrenzt, die durch ein erstes abnehmbares Verschlusselement (6) verschlossen ist, und
(b) ein Pflaster (11) nach einem der vorhergehenden Ansprüche, das so im Inneren des Kompartiments (3) angeordnet ist, dass es heraus genommen werden kann, wobei das Pflaster (11) die Form des Kompartiments (3) aufweist und erhalten wird, indem die Zusammensetzung (P) in das Kompartiment (3) gegossen wird.

22. Einheit nach Anspruch 21, **dadurch gekennzeichnet, dass** die Schale (2) durch Thermoformen oder Spritzgießen dünnwandig gebildet wird.

23. Einheit nach Anspruch 22, **dadurch gekennzeichnet, dass** die Schale (2) aus einem thermoplastischen Stoff, der unter Polyethylenen oder Polypropylenen ausgewählt ist, oder aus einem Komplex von thermoplastischen Stoffen, wie Polyethylenterephthalat/ Aluminium / Polyethylen, oder aus Elastomeren und insbesondere Siliconelastomeren gebildet wird.

24. Einheit nach einem der Ansprüche 21 bis 23, **dadurch gekennzeichnet, dass** die Matrix in dem Kompartiment (3) geformt wird, indem die Zusammensetzung (P) durch die erste Öffnung (5) gegossen wird.

25. Einheit nach einem der Ansprüche 21 bis 24, **dadurch gekennzeichnet, dass** die Schale (2) um die gesamte erste Öffnung (5) herum einen Rand (7) bildet, so dass das erste Verschlusselement (6) insbesondere durch Kleben, Heißsiegeln oder Schweißen daran befestigt werden kann.

26. Einheit nach einem der Ansprüche 21 bis 25, **dadurch gekennzeichnet, dass** das erste Verschlusselement (6) aus einem Deckel besteht, der von einem metallischen oder thermoplastischen Stoff, wie einem Polyethylen oder einem Polypropylen, oder einem Komplex solcher Stoffe gebildet wird.

27. Einheit nach einem der Ansprüche 21 bis 26, **dadurch gekennzeichnet, dass** sie mindestens eine zweite Öffnung (20) aufweist, die in dem Boden (4) angebracht ist, wobei die Matrix in dem Kompartiment (3) geformt wird, indem die Zusammensetzung (P) durch die zweite Öffnung (20) gegossen wird, wobei die zweite Öffnung (20) nach dem Befüllen des Kompartiments verschlossen wird.

28. Einheit nach Anspruch 27, **dadurch gekennzeichnet, dass** die zweite Öffnung (20) mit einem Deckel (23) verschlossen wird, der um die zweite Öffnung herum angeklebt oder angeschweißt wird.

29. Einheit nach einem der Ansprüche 21 bis 28, **dadurch gekennzeichnet, dass** eine Lage (12) angrenzend an eine erste Seite (22) des Pflasters (11), die dem Boden (4) des Kompartiments gegenüber liegt, oder angrenzend an eine zweite Seite (21) des Pflasters, die an den Boden (4) des Kompartiments (3) angrenzt, angeordnet ist oder in einer Position zwischen der ersten (22) und der zweiten Seite (21) in die Matrix eingebettet ist.

## Claims

1. Cosmetic, pharmaceutical or dermatological patch comprising a matrix formed from a composition (P) containing, in an aqueous phase, a hydrophilic gelling system formed from the association of gellan gum with at least one other hydrocolloid.

2. Cosmetic patch according to Claim 1, **characterized in that** the gelling system represents less than 20% of the total weight of the composition (P).

3. Patch according to Claim 1 or 2, **characterized in that** the gellan gum is present in an amount of at least 0.5% of the total weight of the composition (P).

4. Patch according to any one of Claims 1 to 3, **characterized in that** the gellan gum is present in an amount ranging from 2% to 15% and preferably from 2 to 6% of the total weight of the composition (P).

5. Patch according to any one of Claims 1 to 4, **characterized in that** the hydrocolloid associated with the gellan gum is chosen from the group formed by: cellulose or its derivatives; seaweed extracts; seed extracts; plant exudates; microorganism exudates, such as xanthan gum; fruit extracts; gelling agents of animal origin; synthetic water-soluble gelling polymers; POLYCARE®; silicon derivatives; and their mixtures.

6. Patch according to any one of Claims 1 to 5, **characterized in that** the hydrocolloid associated with the gellan gum is chosen from the group formed by xanthan gum, cellulose derivatives, carob gum, a modified guar gum and their mixtures.

7. Patch according to any one of the preceding claims, **characterized in that** the hydrocolloid associated with the gellan gum is present in an amount ranging from 0.5 to 10% and preferably from 0.5 to 5% of the total weight of the composition (P).

8. Patch according to any one of the preceding claims, **characterized in that** the aqueous phase represents from 60 to 97% of the total weight of the composition (P).

9. Patch according to any one of the preceding claims, **characterized in that** it furthermore comprises at least one fatty phase.

10. Patch according to the preceding claim, **characterized in that** the fatty phase is present in an amount ranging up to 30% and preferably from 0.1 to 20% of the total weight of the composition.

11. Patch according to any one of the preceding claims, **characterized in that** the composition (P) contains a solvent chosen from primary alcohols such as ethanol and isopropanol, glycols such as propylene glycol, butylene glycol, dipropylene glycol and diethylene glycol, glycol ethers such as monopropylene, dipropylene or tripropylene glycol alkyl (C₁-C₄)ethers, monoethylene, diethylene or triethylene glycol and their mixtures.

12. Patch according to any one of the preceding claims, **characterized in that** the composition (P) contains at least one active agent having an effect on the skin, this being chosen especially from antioxidants, free-radical scavengers, moisturizers, bleaching agents, liporegulators, anti-acne agents, anti-seborrhoeic agents, anti-ageing agents, softeners, anti-wrinkle agents, keratolytic agents, antiinflammatories, refreshing agents, cicatrizing agents, vascular protecting agents, antibacterials, antifungals, antiperspirants, deodorants, skin conditioners, desensitizing agents, immunomodulators and nourishing agents, or moisture absorbers (cotton, polyacrylate) or sebum absorbers (Orgasol).

13. Patch according to Claim 12, **characterized in that** the composition (P) includes at least one water-soluble active agent chosen from the following compounds: ascorbic acid and its biologically compatible salts, enzymes, antibiotics, components having a tautening effect, α-hydroxy acids and their salts, hydroxylated polyacids, sucroses and their derivatives, urea, amino acids, oligopeptides, water-soluble plant and yeast extracts, protein hydrolysates, hyaluronic acid, mucopolysaccharides, vitamins B₂, B₆, H and PP, panthenol, folic acid, acetylsalicylic acid, allantoin, glycyrrhetic acid, kojic acid and hydroquinone.

14. Patch according to Claim 12, **characterized in that** the composition (P) includes at least one liposoluble compound chosen from the following compounds: D-α-tocopherol, DL-α-tocopherol, D-α-tocopherol acetate, DL-α-tocopherol acetate, ascorbyl palmitate, vitamin F and vitamin F glycerides, vitamins D, vitamin D₂, vitamin D₃, retinol, retinol esters, retinol palmitate, retinol propionate, β-carotene, D-panthenol, farnesol, farnesyl acetate; jojoba and blackcurrant oils rich in essential fatty acids; keratolytic agents such as salicylic acid, its salts and its esters, 5-(n-octanoyl)salicylic acid and its esters, alkyl esters of α-hydroxy acids such as citric acid, lactic acid, glycolic acid; asiatic acid, madecassic acid, asiaticoside, total extract of centella asiatica, β-glycyrrhetinic acid, α-bisabolol, ceramides such as 2-oleoylamino-1,3-octadecane; phytanetriol, milk sphingomyelin, phospholipids of marine origin, rich in polyunsaturated essential fatty acids, ethoxyquin; extract of rosemary, extract of melissa, quercetin, extract of dried microalgae and steroidal antiinflammatories.

15. Patch according to any one of Claims 1 to 14, **characterized in that** the composition (P) contains at least one setting retarder compound, especially in the form of at least one salt.

16. Patch according to any one of the preceding claims, **characterized in that** the matrix is coloured so as to allow the impurities or residues taken out of the skin during application and/or removal of the patch to be seen.

17. Patch according to Claim 16, **characterized in that** the matrix is coloured by the incorporation of synthetic, mineral or organic pigments.

18. Patch according to any one of Claims 1 to 17, **characterized in that** the patch also includes a mesh (12), especially in the form of a net, a woven fabric or a nonwoven fabric or a perforated film.

19. Patch according to any one of the preceding claims, **characterized in that** it comprises a support on which the said composition is coated, the support, after coating, being cut to the desired shape.

20. Patch according to Claim 19, **characterized in that** the support consists especially of a woven or nonwoven fabric or a perforated plastic film.

21. Assembly (1), which comprises:
a) a shell (2) defining at least one compartment (3) having a bottom (4) and one edge of which delimits, in a face on the opposite side from the said bottom, a first opening (5) closed off by a first removable closure element (6); and
b) a patch (11) according to any one of the preceding claims, removably placed inside the compartment (3), the said patch (11) having the shape of the compartment (3) and obtained by pouring the said composition (P) into the said compartment (3).

22. Assembly according to Claim 21, **characterized in that** the shell (2) is formed by thin-wall injection moulding or thermoforming.

23. Assembly according to Claim 22, **characterized in that** the shell (2) is formed from a thermoplastic, chosen from polyethylenes or polypropylenes, or from a complex of thermoplastics, such as polyethylene terephthalate/aluminium/polyethylene, or elastomers, especially silicone elastomers.

24. Assembly according to any one of Claims 21 to 23, **characterized in that** the matrix is moulded in the said compartment (3) by pouring in the said composition (P) via the said first opening (5).

25. Assembly according to any one of Claims 21 to 24, **characterized in that** the shell (2) forms a rim (7) all around the said first opening (5) so as to allow the first closure element (6) to be fixed, especially by bonding, heat-sealing or welding.

26. Assembly according to any one of Claims 21 to 25, **characterized in that** the first closure element (6) consists of a cover formed from a metallic material or a thermoplastic, such as a polyethylene or a polypropylene, or from a complex of such materials.

27. Assembly according to any one of Claims 21 to 26, **characterized in that** it comprises at least one second opening (20) made in the said bottom (4), the said matrix being moulded in the compartment (3) by pouring in the said composition (P) via the said second opening (20), the said second opening (20) being closed off after the compartment has been filled.

28. Assembly according to Claim 27, **characterized in that** the said second opening (20) is closed off by a cover (23), bonded or welded around the said second opening.

29. Assembly according to any one of Claims 21 to 28, **characterized in that** a mesh (12) is placed near a first face (22) of the patch (11), on the opposite side from the bottom (4) of the compartment, or near a second face (21) of the patch, adjacent to the bottom (4) of the compartment (3), or embedded in the matrix in a position lying between the first face (22) and the second face (21).
